Europäisches Patentamt

European Patent Office

Office européen des brevets

(11) Publication number: **0 036 683**
**B1**

(12) **EUROPEAN PATENT SPECIFICATION**

(45) Date of publication of patent specification: **14.03.84**

(21) Application number: **81200244.2**

(22) Date of filing: **02.03.81**

(51) Int. Cl.³: **C 01 B 33/28,**
**B 01 J 29/28,**
**C 07 C 15/00, C 07 C 2/00**

(54) Process for the preparation of crystalline aluminium silicates, crystalline aluminium silicates so prepared and process for the production of an aromatic hydrocarbon mixture.

(30) Priority: **18.03.80 NL 8001596**

(43) Date of publication of application:
**30.09.81 Bulletin 81/39**

(45) Publication of the grant of the patent:
**14.03.84 Bulletin 84/11**

(84) Designated Contracting States:
**BE DE FR GB IT NL**

(56) References cited:
**EP - A - 0 018 089**
**DE - A - 2 049 755**
**FR - A - 2 090 674**
**FR - A - 2 289 444**
**FR - A - 2 289 445**
**GB - A - 1 161 974**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Post, Martin Franciscus Maria**
**Badhuisweg 3**
**NL-1031 CM Amsterdam (NL)**

(74) Representative: **Puister, Antonius Tonnis, Mr. et al,**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 036 683**

Process for the preparation of crystalline aluminium silicates, crystalline aluminium silicates so
prepared and process for the production of an aromatic hydrocarbon mixture

The invention relates to a process for the preparation of crystalline aluminium silicates with improved catalytic properties.

Monoolefins with at most four carbon atoms in the molecule ($C_4^-$ monoolefins) and hydrocarbon mixtures consisting of more than 75%w $C_4^-$ monoolefins can be converted into aromatic hydrocarbon mixtures using certain crystalline aluminium silicates as the catalyst. The said crystalline silicates are characterized in that after calcination during one hour in air at 500°C they have the following properties:

(a) an X-ray powder diffraction pattern showing as the strongest lines the four lines given in Table A,

(b) in the formula representing the composition of the silicate, expressed in moles of the oxides, the $Al_2O_3/SiO_2$ molar ratio (for the sake of brevity further designated m in this patent application) is less than 0.1.

TABLE A

| d(Å) | Relative intensity |
|---|---|
| 11.1 ±0.2 | VS |
| 10.0 ±0.2 | VS |
| 3.84±0.07 | S |
| 3.72±0.06 | S |

wherein the letters used have the following meanings: VS=very strong; S=strong.

As is known from United States patent specification No. 3,702,886 the silicates concerned may be prepared starting from an aqueous mixture which contains the following compounds; one or more compounds of an alkali metal (M), one or more quaternary alkyl-ammonium compounds ($R_4NX$), one or more silicon compounds with a high $SiO_2$ content and one or more aluminium compounds. In this patent application the term silicon compounds with a high $SiO_2$ content is meant to denote: silicon compounds which, after drying at 120°C and calcining at 500°C, yield a product with an $SiO_2$ content of more than 90%w. The preparation of the crystalline silicates is effected by maintaining the aqueous mixture at elevated temperature until the crystalline silicate has been formed, separating this silicate from the mother liquor and calcining it. In the aqueous mixture from which the silicates are prepared, the various compounds should be present in the following ratios, expressed in moles of the oxides:

$$M_2O: \quad SiO_2 < 0.23,$$
$$(R_4N)_2O: \quad SiO_2 < 0.34,$$
$$H_2O: \quad SiO_2 < 225, \text{ and}$$
$$Al_2O_3: \quad SiO_2 < 0.1.$$

In the European patent application 0018089 filed before the present application the preparation of zeolite ZSM-12 is described using a mixture of an alkali metal oxide, and oxide of silicon, an oxide of aluminium, a methyltriethyl-ammonium salt and water.

The molar ratio of the oxide of methyltriethyl-ammonium to silicon dioxide is below 0.145 and the molar ratio of water to silicon dioxide is between 2.5 and 140.

In an investigation by the Applicant concerning the use of the above-mentioned ZSM5 type silicates as catalysts for the preparation of aromatic hydrocarbon mixtures from $C_4^-$ monoolefins and from hydrocarbon mixtures consisting of more than 75%w $C_4^-$ monoolefins, it has been found that the stability of the aromatics selectivity of these catalysts is greatly determined by the $(R_4N)_2O/SiO_2$ molar ratio in the aqueous mixture from which the crystalline silicates are prepared. It has been found that when an $H_2O/SiO_2$ molar ratio of more than 12 is employed in the aqueous mixture, which is a usual ratio in the preparation of these silicates, the stability of the aromatics selectivity of the catalysts falls according as a lower $(R_4N)_2O/SiO_2$ molar ratio is employed in the aqueous mixture and that, when an $(R_4N)_2O/SiO_2$ molar ratio of less than 0.15 is employed, catalysts with an unacceptably low stability of the aromatics selectivity are obtained. As the quaternary alkyl-ammonium compounds are rather expensive in comparison with the other reaction components in the aqueous mixture, a method is needed for the preparation of crystalline silicates which show a high stability in aromatics selectivity when used for the above-mentioned catalytic application, without a high $(R_4N)_2O/SiO_2$ molar ratio being required in the preparation. Further investigation by the Applicant concerning this subject has shown that crystalline silicates which show a high stability of the aromatics selectivity in the above-mentioned catalytic application, can be prepared from an aqueous mixture, in which the $(R_4N)_2O/SiO_2$ molar ratio lies between 0.07 and 0.15, provided that the $H_2O/SiO_2$ molar ratio in the aqueous mixture lies between 8 and 12. Use of the above-mentioned unusually low $H_2O/SiO_2$ molar ratio in the aqueous mixture from which the crystalline silicates are prepared, has the additional advantage that the yield of

2

crystalline silicate per unit of reactor volume is extremely high. The preparation of crystalline aluminium silicates from an aqueous mixture in which the $(R_4N)_2O/SiO_2$ and $H_2O/SiO_2$ molar ratios show the above-mentioned low values, is novel.

The present patent application therefore relates to a novel process for the preparation of crystalline aluminium silicates having the properties mentioned under (a) and (b), in which process an aqueous mixture that contains the following compounds: one or more compounds of an alkali metal (M), one or more quaternary alkylammonium compounds ($R_4NX$), one or more silicon compounds with a high $SiO_2$ content and one or more aluminium compounds, in which mixture the various compounds are present in the following ratios, expressed in moles of the oxides:

$$M_2O: \quad SiO_2 = 0.01\text{---}0.35,$$
$$(R_4N)_2O: \quad SiO_2 = 0.07\text{---}0.15,$$
$$H_2O: \quad SiO_2 = 8\text{---}12, \text{ and}$$
$$Al_2O_3: \quad SiO_2 < 0.1,$$

is maintained at elevated temperature until the crystalline silicate has been formed, and this silicate is separated from the mother liquor and calcined.

The silicates prepared according to the invention have been defined, inter alia, with reference to the X-ray powder diffraction pattern. This pattern should show as the strongest lines the four lines given in Table A. The complete X-ray powder diffraction pattern of a typical example of a silicate prepared according to the invention is shown in Table B.

TABLE B

| d(Å) | Relative intensity | d(Å) | Relative intensity |
|------|------|------|------|
| 11.1 | 100 | 4.00 | 3 |
| 10.0 | 70 | 3.84 | 57 |
| 8.93 | 1 | 3.72 | 31 |
| 7.99 | 1 | 3.64 | 10 |
| 7.42 | 1 | 3.44 | 5 |
| 6.68 | 7 | 3.34 | 3 |
| 6.35 | 11 | 3.30 | 5 |
| 5.97 | 18 | 3.25 | 2 |
| 5.70 | 7 | 3.05 | 5 |
| 5.56 | 10 | 2.98 | 12 |
| 5.35 | 2 | 2.96 | 3 |
| 4.98 | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |

The preparation of the silicates may be carried out both at atmospheric pressure and at elevated pressure. If reaction temperatures are used which are above the boiling point of the mixture, it is preferred to work under autogenous pressure in an autoclave. The silicates are preferably prepared by maintaining the mixture for at least four hours at a temperature between 90 and 300°C and in particular at a temperature between 125 and 175°C. After the silicates have been formed, the crystals are separated from the mother liquor, for instance by filtering, decanting or centrifuging. The crystal mass is subsequently washed with water and finally dried and calcined.

Examples of suitable compounds that may be used in the preparation of the silicates according to the invention are nitrates, carbonates, hydroxides and oxides of alkali metals; quaternary alkylammonium bromides and hydroxides; amorphous solid silicas, silica sols, silica gels and silicic acid; aluminium hydroxide, sodium aluminate, aluminium sulphate and gamma alumina. In the preparation of the silicates according to the invention it is preferred to start from an aqueous mixture in which M is present in a sodium compound and $R_4NX$ is a tetrapropylammonium compound. In the preparation of the silicates according to the invention it is further preferred to start from an aqueous mixture in which the aluminium and silicon compounds, expressed in moles of the oxides, are present in a ratio greater than 0.002 and in particular greater than 0.0025.

Silicates prepared according to the invention may, for instance, be used as adsorbent and extractant, as drying agent, as ion exchanger and as catalyst or catalyst carrier in various catalytic processes, in particular the catalytic preparation of aromatic hydrocarbons from acyclic organic compounds. If the aim is to use the silicates prepared according to the invention as catalyst or catalyst carrier, it is preferred to previously reduce the alkali metal content of these silicates to less than 0.1%w and in particular to less than 0.01%w. The reduction of the alkali metal content of the silicates can very suitably be carried out by contacting them once or several times with an aqueous solution containing

O 036 683

ammonium ions. From the $NH_4^+$ silicates thus obtained the $H^+$ silicates can be prepared by calcination. When they are used as catalyst the crystalline aluminium silicates may, if desired, be combined with a binder material such as bentonite or kaolin.

As mentioned hereinbefore, an important application of the silicates prepared according to the invention is their use as catalyst in the preparation of an aromatic hydrocarbon mixture from a $C_4^-$ monoolefin or from a hydrocarbon mixture consisting of more than 75%w $C_4^-$ monoolefins. Suitable $C_4^-$ monoolefins are: ethene, propene, butene and isobutene. If the starting material is a hydrocarbon mixture containing, in addition to one or more $C_4^-$ monoolefins, one or more other hydrocarbons, these other hydrocarbons may be, inter alia paraffins, diolefins or $C_5^+$ monoolefins. A preferred starting material is a $C_3$ or $C_4$ monoolefin or a hydrocarbon mixture consisting substantially of one or more of these monoolefins. A very suitable feed is a hydrocarbon mixture consisting substantially of $C_3$ and/or $C_4$ monoolefins obtained as by-product in the catalytic or thermal cracking of hydrocarbons, in particular in the thermal cracking of hydrocarbons for the preparation of ethene. The process is preferably carried out at a temperature of 300—550°C and in particular 350—500°C, a pressure of 3—20 bar and in particular of 5—15 bar and a space velocity of 1—20 $g \cdot g^{-1} \cdot h^{-1}$ and in particular of 2—10 $g \cdot g^{-1} \cdot h^{-1}$. If desired, the process may be carried out in the presence of hydrogen. In the process it is preferred to use crystalline aluminium silicates prepared according to the invention, for which holds that $0.0075 \geqslant m \geqslant 0.0030$ and which have a crystallite size of at most 500 nm.

Crystalline aluminium silicates prepared according to the invention are also very suitable for use as catalyst component in the preparation of aromatic hydrocarbon mixtures from $H_2/CO$ mixtures. This conversion is preferably carried out by contacting an $H_2/CO$ mixture having an $H_2/CO$ molar ratio below 1.0 with a mixture of two catalysts of which one has the capability of catalysing the conversion of an $H_2/CO$ mixture into acyclic oxygen-containing hydrocarbons and the other is the crystalline aluminium silicate. $H_2/CO$ mixtures having an $H_2/CO$ molar ratio below 1.0 can very suitably be prepared by steam gasification of coal at a temperature of 900—1500°C and a pressure of 10—50 bar. In respect of the catalysts having the capability of catalysing the conversion of an $H_2/CO$ mixture into acyclic oxygen-containing hydrocarbons, preference is given to catalysts having the capability of converting an $H_2/CO$ mixture into substantially methanol and/or dimethyl ether. Particular preference is given to catalysts containing the metal combination Zn—Cr. The conversion of the $H_2/CO$ mixture into an aromatic hydrocarbon mixture is preferably carried out at a temperature of 200—500°C and in particular of 250—450°C, a pressure of 1—150 bar and in particular of 5—100 bar and a space velocity of 50—5000 and in particular of 300—3000 Nl gas/l catalyst/h. The above-described conversion using a mixture of a crystalline aluminium silicate prepared according to the invention and a catalyst having the capability of catalysing the conversion of an $H_2/CO$ mixture into acyclic oxygen-containing hydrocarbons, can also very suitably be used as the first step in a two-step process for the conversion of an $H_2/CO$ mixture having an $H_2/CO$ molar ratio below 1.0 into an aromatic hydrocarbon mixture. In this case, at least the $C_2^-$ fraction of the reaction product from the first step is contacted in a second step with a catalyst containing one or more metal components having catalytic activity for the conversion of an $H_2/CO$ mixture into acyclic hydrocarbons, which metal components have been chosen from the group formed by cobalt, nickel and ruthenium, on the understanding that, if the feed for the second step has an $H_2/CO$ molar ratio of less than 1.5, water is added to this feed and that in the second step a bifunctional catalyst combination is used which contains, in addition to the metal components having catalytic activity for the conversion of an $H_2/CO$ mixture into acyclic hydrocarbons, also one or more metal components having catalytic activity for the conversion of an $H_2/CO$ mixture into an $H_2/CO_2$ mixture.

The invention will now be explained with reference to the following example.

Example

Four crystalline aluminium silicates (silicates 1—4) were prepared by heating mixtures of NaOH amorphous silica, $(C_3H_7)_4NOH$ and $NaAlO_2$ for 24 hours in water in an autoclave at 150°C. After the reaction mixtures had cooled down, the silicates formed were filtered off, washed with water until the pH of the wash water was about 8, dried at 120°C and calcined at 500°C. Silicates 1—4 had the following properties:

(a) an X-ray powder diffraction pattern substantially equal to the one given in Table B;
(b) a value for m as given in Table C.

TABLE C

| Silicate No. | m |
| --- | --- |
| 1 | 0.0059 |
| 2 | 0.0053 |
| 3 | 0.0063 |
| 4 | 0.0056 |

The amorphous silica used in the preparation of silicates 1—4 led, after drying at 120°C and calcining at 500°C, to the formation of a product consisting of 99.97%w $SiO_2$.

4

The molar composition of the aqueous mixtures from which silicatess1—4 were prepared can be represented as follows: 25 $SiO_2$. 0.125 $Al_2O_3$. 1 $Na_2O$ . x$[(C_3H_7)_4N]_2O$ . y$H_2O$, wherein x and y have the values given in Table D. Table D also shows the $H_2O/SiO_2$ and $[(C_3H_7)_4N]_2O/SiO_2$ molar ratios used in the aqueous mixtures.

TABLE D

| Silicate No. | x | y | $H_2O/SiO_2$ | $[(C_3H_7)_4N]_2O/SiO_2$ |
|---|---|---|---|---|
| 1 | 2.5 | 250 | 10 | 0.10 |
| 2 | 1.5 | 250 | 10 | 0.06 |
| 3 | 2.5 | 450 | 18 | 0.10 |
| 4 | 1.5 | 450 | 18 | 0.06 |

Silicates I—IV were prepared from silicates 1—4, respectively, by boiling the materials calcined at 500°C with 1.0 molar $NH_4NO_3$ solution, washing with water, boiling again with 1.0 molar $NH_4NO_3$ solution and washing, drying at 120°C and calcining at 500°C.

Silicates I—IV were tested as catalyst for the preparation of an aromatic hydrocarbon mixture from isobutene. The test was carried out in a 50 ml reactor containing a fixed catalyst bed having a volume of 5 ml consisting of the silicate concerned. Isobutene was conducted over the catalyst at a temperature of 400°C, a pressure of 10 bar, a space velocity of 3.4 g isobutene/g silicate/h and an $H_2$/isobutene molar ratio of 5:1. The results of these experiments are listed in Table E. The aromatics selectivities are given in this table as yield in aromatics in %w, based on isobutene feed.

TABLE E

| Experiment No. | 1 | 2 | 3 | 4 |
|---|---|---|---|---|
| Silicate No. | I | II | III | IV |
| | | | | |
| Aromatics selectivity %w | | | | |
| after 1 day | 25 | 23 | 24 | 23 |
| after 10 days | 22 | 12 | 8 | 4 |

Of the silicates listed in Table D only silicate 1 was prepared according to the invention. Silicates 2—4 are outside the scope of the invention. They have been included in the patent application for comparison. Of the experiments listed in Table E, only experiment 1 was carried out using a catalyst prepared according to the invention. In this experiment a high aromatics selectivity was reached. Experiments 2—4 are outside the scope of the invention and have been included for comparison. In these experiments an unacceptably rapid fall of the aromatics selectivity was observed.

## Claims

1. A process for the preparation of crystalline aluminium silicates which, after calcination for 1 hour in air at 500°C, have the following properties:

(a) an X-ray powder diffraction pattern showing as the strongest lines the four lines given in Table A.

TABLE A

| d(Å) | Relative intensity |
|---|---|
| 11.1 ±0.2 | VS |
| 10.0 ±0.2 | VS |
| 3.84±0.07 | S |
| 3.72±0.06 | S |

wherein the letters used have the following meanings: VS=very strong; S=strong, and

(b) in the formula representing the composition of the silicate, expressed in moles of the oxides, the $Al_2O_3/SiO_2$ molar ratio (m) is less than 0.1, characterized in that an aqueous mixture containing the following compounds: one or more compounds of an alkali metal (M), one or more quaternary alkylammonium compounds ($R_4NX$), one or more silicon compounds which, after drying at 120°C and calcining at 500°C, yield a product with an $SiO_2$ content of more than 90%w and one or more aluminium compounds, in which mixture the various compounds are present in the following molar ratios, expressed in moles of the oxides:

$$M_2O: \quad SiO_2=0.01—0.35,$$
$$(R_4N)_2O: \quad SiO_2=0.07—0.15,$$
$$H_2O: \quad SiO_2=8—12, \text{ and}$$
$$Al_2O_3: \quad SiO_2<0.1,$$

is maintained at elevated temperature until the crystalline silicate has been formed, and in that this silicate is subsequently separated from the mother liquor and calcined.

2. A process according to claim 1, characterized in that as $R_4NX$ compound use is made of a tetrapropylammonium compound and as alkali metal compound of a sodium compound.

3. A process according to claim 1 or 2, characterized in that the mixture is maintained for at least four hours at a temperature between 90 and 300°C.

4. A process according to any one of claims 1—3, characterized in that the starting material is an aqueous mixture in which the aluminium and silicon compounds, expressed in moles of the oxides, are present in a ratio greater than 0.002.

5. Crystalline aluminium silicates prepared according to a process as claimed in any one of claims 1—4, characterized in that their alkali metal content has been reduced to less than 0.1%w.

6. Use of the product obtained by a process as claimed in any one of claims 1—4 as a catalyst for the production of aromatic hydrocarbons.

## Revendications

1. Un procédé de préparation de silicates d'aluminium cristallins qui, après calcination pendant 1 heure dans l'air à 500°C, présentent les propriétés suivants:

(a) un spectre de diffraction de rayon X en poudre présentant comme raies les plus fortes les quatre raies données dans le Tableau A.

### TABLEAU A

| d(Å) | Intensité relative |
|---|---|
| 11,1 ±0,2 | TF |
| 10,0 ±0,2 | TF |
| 3,84±0,07 | F |
| 3,72±0,06 | F |

où les lettres utilisées ont la signification suivante: TF=très forte; F=forte, et

(b) dans la formule représentant la composition du silicate, exprimée en moles d'oxydes, le rapport molaire $Al_2O_3/SiO_2$ (m) est inférieur à 0,1, caractérisé en ce qu'un mélange aqueux contenant les composés suivants: un ou plusieurs composés d'un métal alcalin (M), un ou plusieurs composés d'alkylammonium quaternaire ($R_4NX$), un ou plusieurs composés de silicium qui, après séchage à 120°C et calcination à 500°C, donnent un produit avec une teneur en $SiO_2$ de plus de 90% en poids et un ou plusieurs composés d'aluminium, les divers composés étant présents, dans ce mélange, dans les rapports molaires suivants, exprimés en moles d'oxydes:

$$M_2O: \quad SiO_2=0,01—0,35,$$
$$(R_4N)_2O: \quad SiO_2=0,07—0,15,$$
$$H_2O: \quad SiO_2=8—12, \text{ et}$$
$$Al_2O_3: \quad SiO_2<0,1,$$

est maintenu à une température élevée jusqu'à ce que le silicate cristallin ait été formé, et en ce que ce silicate est ensuite séparé de la solution mère et calciné.

2. Un procédé selon la revendication 1, caractérisé en ce qu'on utilise comme composé $R_4NX$ un composé de tétrapropylammonium et comme composé de métal alcalin un composé de sodium.

3. Un procédé selon les revendications 1 ou 2, caractérisé en ce que le mélange est maintenu pendant au moins quatre heures à une température comprise entre 90 et 300°C.

4. Un procédé selon l'importe laquelle des revendications 1—3, caractérisé en ce que le matériau de départ est un mélange aqueux dans lequel les composés d'aluminium et de silicium, exprimés en moles d'oxydes, sont présents dans un rapport supérieur à 0,002.

5. Des silicates d'aluminium cristallins préparés selon un procédé comme revendiqué dans n'importe laquelle des revendications 1—4, caractérisés en ce que leur teneur en métal alcalin a été réduite à moins de 0,1% en poids.

6. Une utilisation du produit obtenu par un procédé comme revendiqué dans n'importe laquelle des revendications 1—4 comme catalyseur pour la production d'hydrocarbones aromatiques.

## Patentansprüche

1. Ein Verfahren zur Herstellung von kristallinen Aluminiumsilikaten, welche nach einstündiger Calcinierung an Luft bei 500°C die folgenden Eigenschaften aufweisen:

a) ein Röntgenpulverdiagramm, welches als stärkste Linien die in Tabelle A angegebenen Linien hat:

**0 036 683**

| d(Å) | Relative Intensität |
|---|---|
| 11,1 ±0,2 | VS |
| 10,0 ±0,2 | VS |
| 3,84±0,07 | S |
| 3,72±0,06 | S |

in welcher die verwendeten Buchstaben die folgende Bedeutung haben: VS=sehr stark, S=stark, und

b) in der die Zusammensetzung des Silikats wiedergebenden Formel, ausgedrückt un Molen der Oxide, das molare Verhältnis (m) $Al_2O_3/SiO_2$ kleiner als 0,1 ist, dadurch gekennzeichnet, daß eine wässrige Mischung, welche die folgenden Verbindungen enthält: eine oder mehrere Verbindungen eines Alkalimetalls (M), eine oder mehrere quaternäre Alkylammoniumverbindungen ($R_4NX$), eine oder mehrere Siliciumverbindungen, welche nach Trocknen bei 120°C und Calcinieren bei 500°C ein Produkt mit einem $SiO_2$-Gehalt von mehr als 90 Gewichtsprozent liefern, und eine oder mehrere Aluminiumverbindungen, in welcher Mischung die verschiedenen Verbindungen in den folgenden molaren Verhältnissen, ausgedrückt in Molen der Oxide, enthalten sind:

$$M_2O: \quad SiO_2=0,01\text{---}0,35,$$
$$(R_4N)_2O: \quad SiO_2=0,07\text{---}0,15,$$
$$H_2O: \quad SiO_2=8\text{---}12, \text{ und}$$
$$Al_2O_3: \quad SiO_2<0,1,$$

so lange auf erhöhter Temperatur gehalten wird, bis sich das kristalline Silikat gebildet hat, und daß dieses Silikat anschließend von der Mutterlauge abgetrennt und calciniert wird.

2. Ein Verfahren gemäß Anspruch 1, dadurch gekennzeichnet, daß als Verbindung $R_4NX$ eine Tetrapropylammoniumverbindung und als Alkalimetallverbindung eine Natriumverbindung verwendet wird.

3. Ein Verfahren gemäß Anspruch 1 oder 2, dadurch gekennzeichnet, daß die Mischung mindestens 4 Stunden lang auf einer Temperatur zwischen 90 und 300°C gehalten wird.

4. Ein Verfahren gemäß irgendeinem der Ansprüche 1 bis 3, dadurch gekennzeichnet, daß das Ausgangsmaterial eine wässrige Mischung ist, in welcher die Aluminium- und Siliciumverbindungen, ausgedrückt in Molen der Oxide, in einem Verhältnis größer als 0,002 vorhanden sind.

5. Kristalline Aluminiumsilikate, hergestellt nach einem Verfahren wie in irgendeinem der Ansprüche 1 bis 4 beansprucht, dadurch gekennzeichnet, daß ihr Alkalimetallgehalt auf weniger als 0,1 Gewichtsprozent verringert worden ist.

6. Verwendung des Produktes, erhalten durch ein Verfahren wi in irgendeinem der Ansprüche 1 bis 4 beansprucht, als ein Katalysator für die Herstellung von aromatischen Kohlenwasserstoffen.